# EUROPEAN PATENT APPLICATION

(11) **EP 2 042 169 A1**
(43) Date of publication of application: **01.04.2009**
(21) Application number: 07117285.2
(22) Date of filing: 26.09.2007
(51) Int. Cl.: A61K 31/18, A61K 9/14, A61P 13/08, A61P 13/10

(54) **Controlled release tamsulosin hydrochloride tablets and a process of making them**

(71) Applicant: LEK Pharmaceuticals d.d., 1526 Ljubljana (SI)
(72) Inventor: The designation of the inventor has not yet been filed
(74) Representative: Kröger, Bernd

(57) **Abstract**

The present invention relates to a controlled release preparation of tamsulosin hydrochloride tablets comprising hydroxypropylcellulose and polyethylene oxide, and the process for making such tablets.

## Description

### Field of the invention

The present invention belongs to the field of pharmaceutical technology, more specifically to the field of oral pharmaceutical compositions and relates to a controlled release preparation of tamsulosin hydrochloride in the form of tablets capable of releasing tamsulosin for a prolonged time and the process for making such tablets.

The invented tablets are capable of prolonged release of the active ingredient and at the same time are prepared by a technological procedure which enables the preparation of tablets with a low dose of active ingredient and a good content uniformity. Furthermore the technological procedure of this invention solves the problem of segregation of different phases (granulated phase and other excipients) during the compression of low dose tamsulosin hydrochloride tablets.

### Background of the invention

Tamsulosin is a selective antagonist of α_{1A} and α_{1D} adrenergic receptors. It is indicated for the treatment of benign prostatic hyperplasia. By selective and competitive binding to α₁ postsynaptic receptors, it relaxes smooth muscles in the prostate and the urinary bladder neck thereby increasing the urinary flow, facilitating urination and improving other symptoms of benign prostatic hyperplasia.

Tamsulosin from immediate release formulations is rapidly absorbed and plasma concentrations increase quickly. By developing modified release pharmaceutical formulations, an important step in improving the tolerance and prolonging activity of the active substance can be made. With modified release formulations, the likelihood of causing vasodilatation and related cardiovascular side effects is diminished.

In the patent literature, different technological approaches for development of controlled release tamsulosin hydrochloride are presented:

EP 661045 discloses a sustained release hydrogel preparation having a gelation index of 70 % or more, based on an additive ensuring penetration of water into the composition and a hydrogel-forming polymer having an average molecular weight of not less than 2,000,000 or a viscosity of not less than 1000 cps measured at 1 % concentration in water at 25°C.

EP 1568361 and EP 1529526 disclose several approaches for sustained release pharmaceutical compositions containing tamsulosin, such as a sustained-release hydrogel-forming one, an osmotic pump type preparation, a gel preparation where a plurality of gums are combined, a multi-layered tablet comprising a geometrically aligned drug layer and release controlling layers, a gastroretentive dosage form using a swelling polymer, a matrix preparation using water-soluble polymers.

EP 1443917 discloses a pharmaceutical tablet with tamsulosin obtainable by a process without the use of a liquid.

EP 1441713 and DE 20221486 disclose a monolithic pharmaceutical tablet, comprising 0.1 - 10 mg of tamsulosin or a pharmaceutically acceptable salt thereof, 10 - 90 wt. % of hydroxypropylmethylcellulose (HPMC), the total tablet mass being 10 - 300 mg.

EP 1205190 discloses a matrix type sustained-release preparation on the basis of polyethylene oxide (i) having a viscosity of 2,000 cP or more as an aqueous 2 % solution at 25°C or (ii) having a viscosity average molecular weight of 2,000,000 or more, said drug and hydrophilic base being dispersed in a matrix of said polyethylene oxide, and yellow ferric oxide and/or red ferric oxide to stabilize the drug release rate of the preparation.

In an article in European Urology Supplements 4 (2005), p.1-4, the advantages of OCAS system in comparison to a classical hydrophilic matrix is described.

EP 1523994 discloses the manufacturing of a controlled release preparation comprising particles comprised of a drug, PEO with molecular mass above 2,000,000 and a specific size controlling agent obtained by sizing. The powder particles obtained by sizing are prepared by re-binding of smaller PEO particles back to bigger PEO particles ensuring a suitable particle size and content uniformity. The purpose of the granulation is to ensure the content uniformity.

EP 1595538 and DE 20221486 disclose a pharmaceutical tablet with tamsulosin or a pharmaceutically acceptable salt thereof wherein the polymeric matrix component is selected from a group comprising a water swellable cellulosic derivative; sodium alginate; acrylates, methacrylates and co-polymers thereof with various comonomers; and polyvinylpyrrolidones and wherein the water swellable cellulosic derivative is carboxymethylcellulose, cellulose acetate, hydroxyethylcellulose, hydroxypropylcellulose, preferably HPMC. Example 4 describes the preparation of a tablet by compaction, milling and tablet compression. The milling step has not been described as being related to content uniformity.

The present invention is thus aimed at preparing a stable formulation of tamsulosin hydrochloride controlled release tablets having a good content uniformity.

The solution proposed by the inventors relates to a granulate preparation which ensures an improved intrabatch content uniformity of prepared tablets due to the reduced segregation of the final mixture during the compression process. The content uniformity is generally solved by the preparation of a granulate as an intermediate step. We have shown that besides the granulation used to ensure a good content uniformity, milling of the prepared granulate can help improving the content uniformity. The addition of a milling step following the wet granulation to improve content uniformity is surprising and is not considered to be a common production practice.

### Summary of the invention

In the first aspect of the present invention, we provide a process for the preparation of the pharmaceutical composition - controlled release tamsulosin hydrochloride tablets - comprising the preparation of the granulate, the milling thereof and tabletting of the milled granulate.

In another aspect, we provide a pharmaceutical composition - controlled release tamsulosin hydrochloride tablets - comprising hydroxypropylcellulose and polyethylene oxide.

In another aspect, we provide a pharmaceutical composition - controlled release tamsulosin hydrochloride tablets - comprising medium viscosity and high viscosity hydroxypropylcellulose, and polyethylene oxide.

In another aspect, we provide a use of such pharmaceutical formulations with tamsulosin hydrochloride for the preparation of a medicament for the prevention or treatment of benign prostatic hyperplasia and other related states or conditions.

### Detailed description of the invention

Controlled release tamsulosin hydrochloride tablets contain a very low amount of the active ingredient. It is known in the common pharmaceutical practice that several problems can arise in the manufacturing of such low dose preparations. On the other hand, prolonged release of such a low amount of the active ingredient is also desirable. Preferably at least 24-hour release is most desirable. To achieve such a release profile, tamsulosin hydrochloride tablets were made on the basis of hydrophilic matrix tablets. A combination of two hydrogel forming polymers was selected. The first one was a high molecular weight (high viscosity) hydroxypropylcellulose (HPC) and the second one was a high molecular weight (high viscosity) polyethylene oxide (PEO). Both polymers play a synergistic role in a formation of an effective prolonged release formulation, capable of releasing low amounts of tamsulosin active ingredient for a prolonged time.

High molecular weight (high viscosity) hydroxypropylcellulose is known to effectively sustain the release of drugs. Its disadvantage is slow swelling in water therefore higher release rates of active ingredients from incompletely hydrated matrix systems are expected in the first few hours after ingestion of such tablets (»burst effect«). To the contrary, polyethylene oxide polymers due to their hydrophilic nature swell more rapidly than hydroxypropylcellulose which ensures almost complete hydration of polyethylene oxide polymer chains during the first few hours after the contact of the polymer with water medium. The addition of polyethylene oxide to hydroxypropylcellulose can thus prevent a so-called burst effect and provide a hydrophilic matrix system that is more uniformly hydrated than the hydrophilic matrix from hydroxypropylcellulose alone.

To ensure a good content uniformity and additionally reduce the burst effect of tamsulosin hydrochloride active ingredient from the hydrophilic matrix composed of a combination of hydroxypropylcellulose and polyethylene oxide, wet granulation of tamsulosin hydrochloride with a medium molecular weight (medium viscosity) hydroxypropylcellulose and microcrystalline cellulose (inert filler / diluent) was included in a technological process to prepare controlled release tablets.

The high molecular weight (high viscosity) hydroxypropylcellulose may have viscosity of 2 % aqueous solution at 25°C above 2000 mPa*s or Mr above 500,000; preferably viscosity above 3000 mPa*s or Mr above 700,000; more preferably in the range of 4000-6500 mPa*s or Mr around 850,000. The amounts thereof may comprise 10-90 %, preferably 20-60 %, more preferably 30-40 % of the total mass of the tablet.

The medium molecular weight (medium viscosity) hydroxypropylcellulose may have viscosity of 2 % aqueous solution at 25°C is in the range of 10-2000 mPa*s or Mr in the range of 90,000 - 500,000; preferably viscosity in the range of 100-1000 mPa*s or Mr in the range of 300,000 - 400,000; more preferably viscosity in the range of 150-400 mPa*s or Mr around 370,000. The amounts thereof may comprise comprising 1-50 %, preferably 2-20 %, more preferably 3-5 % of the total mass of the tablet.

Polyethylene oxide may be high molecular weight (high viscosity) polyethylene oxide having viscosity of 1 % aqueous solution at 25°C above 1000 mPa*s or Mr above 2,000,000; preferably viscosity above 5000 mPa*s or Mr above 5,000,000; more preferably viscosity in the range of 7500-10000 mPa*s or Mr around 7,000,000. The amounts thereof may comprise 10-90 %, preferably 15-50 %, more preferably 20-30 % of the total weight of the tablet.

High molecular weight (high viscosity) hydroxypropylcellulose is available in two particle sizes (regular grind: 95 % of particles pass through a US #30 mesh (590 µm) sieve, and 99 % of particles pass through a US #20 mesh (840 µm) sieve). For the preparation of controlled release matrix systems, hydroxypropylcellulose with a smaller particle size is preferable (i.e. Klucel X grades; X grind: 100 % of particles pass through a US #60 mesh (250 µm) sieve, and 80% of particles pass through a US #100 mesh (149 µm) sieve), since smaller particles sustain the release of active ingredients better than larger ones. Unfortunately the classical wet granulation procedure, by which the above mentioned granulate of tamsulosin hydrochloride active ingredient with the medium viscosity hydroxypropylcellulose was prepared, gave a granulate with a larger particle size than that of the high viscosity fine grade hydroxypropylcellulose. After mixing of this granulate with the fine grade hydroxypropylcellulose, a problem occurred. In compression machine, this mixture segregated which resulted in poor intrabatch content uniformity of tablets during the tabletting process from the beginning up to the end. To solve this problem, the classical wet granulation was improved by the final reduction of the size of the granules and we surprisingly found that a change in the granulation procedure gave the granulate with much better characteristics than the previous procedure. The use of the improved granulate in the preparation of the final mixture for compression therefore reduced the segregation in the compression machine and consequently improved the intrabatch content uniformity of prepared tablets.

### The so-called classical granulate was prepared in the following way:

Tamsulosin hydrochloride was mixed with microcrystalline cellulose and medium viscosity hydroxypropylcellulose. This mixture was granulated with water in a high shear granulator. The obtained wet granulate was dried in a fluid bed dryer. During drying, the almost dried granulate was sieved through a sieve with mesh size 1.200 mm to break down larger agglomerates. After the drying was completed, the granulate was sieved through a sieve with mesh size 0.500 mm.

Then the prepared granulate was mixed with polyethylene oxide and other excipients and compressed on a rotary compressing machine.

### The improved granulate was prepared in the following way:

Tamsulosin hydrochloride was mixed with microcrystalline cellulose and medium viscosity hydroxypropylcellulose. This mixture was granulated with water in a high shear granulator. The obtained wet granulate was dried in a fluid bed dryer. During drying, the almost dried granulate was sieved through sieve with mesh size 1.200 mm to break down larger agglomerates. After the drying was completed, the granulate was milled using a hammer mill through a sieve with mesh size 0.500 mm.

Then the prepared granulate was mixed with polyethylene oxide and other excipients and compressed on a rotary compressing machine.

As mentioned above, the second granulate, which was prepared by milling, surprisingly gave much better results in the intrabatch content uniformity of prepared tablets, because of the reduced segregation of the final mixture during compression process. Since milling after wet granulation is not a common practice to improve the content uniformity, the obtained results are unexpected for pharmaceutical researchers dealing with dosage form designing.

The tablet cores can be additionally coated with a conventional coloured coating.

The following examples are offered to illustrate aspects of the present invention and are not intended to limit or define the present invention in any manner.

### EXAMPLE 1

Preparation of controlled release tamsulosin hydrochloride tablets using the classical wet granulation approach

### a) Tablet composition

| | mg / tablet |
|---|---|
| Tamsulosin HCl | 0.40 |
| Microcrystalline cellulose | 62.60 |
| Medium viscosity HPC | 7.00 |
| High viscosity HPC | 87.50 |
| Spray dried lactose | 27.75 |
| High viscosity PEO | 62.50 |
| Butylhydroxy toluene | 0.25 |
| Mg stearate | 1.25 |
| Colloidal silica | 1.25 |
| Total tablet core | 250.00 |

### b) Manufacturing procedure

A triturate of tamsulosin hydrochloride, microcrystalline cellulose and medium viscosity hydroxypropylcellulose in the ratio 1 : 40.25 : 8.75 was prepared by mixing all ingredients in a plastic bag.

The obtained triturate was mixed for 5 min. with the remaining part of microcrystalline cellulose in a high shear granulator GRAL PRO 25, (impeller speed 100 rpm). This mixture was granulated with water (high shear granulator GRAL PRO 25, addition of water 58 ml/min., impeller speed 100 rpm, chopper speed slow). The mixture was additionally granulated (high shear granulator GRAL PRO 25, impeller speed 150 rpm, chopper speed slow, time 5 min.). The wet granulate was dried in a fluid bed dryer (Glatt GPCG 3, inlet air temperature 60 °C). When the temperature of the product reached 45 °C, the granulate was sieved through a sieve with mesh size 1.200 mm to break down larger agglomerates and transferred back to the fluid bed dryer. After the drying was completed, the granulate was sieved through a sieve with mesh size 0.500 mm (Frewitt).

**Particle size distribution (Sieve analysis)**

| Sieve (µm) | Retained above (%) |
|---|---|
| 400 | 11.7 |
| 315 | 8.7 |
| 200 | 10.9 |
| 125 | 9.7 |
| 100 | 8.5 |
| 45 | 36.6 |
| under 45 | 13.9 |

Then the prepared granulate was mixed with other excipients by gradually adding individual excipients (Erweka). After the addition of excipients, the mixture was blended for 5 minutes. The final mixture was compressed on a rotary compressing machine Kilian LX 18.

### c) Characterization of the produced tablets

**Intrabatch content uniformity**

| | mg / tablet | % of declared dose |
|---|---|---|
| Beginning of the tabletting | 0.4431 | 110.8 |
| Middle of the tabletting | 0.3849 | 96.2 |
| End of the tabletting: | 0.3900 | 97.5 |

### EXAMPLE 2

Preparation of controlled release tamsulosin hydrochloride tablets using the invented wet granulation approach

### a) Tablet composition

| | mg / tablet |
|---|---|
| Tamsulosin HCl | 0.40 |
| Microcrystalline cellulose | 62.60 |
| Medium viscosity HPC | 7.00 |
| High viscosity HPC | 87.50 |
| Spray dried lactose | 18.75 |
| Microcrystalline cellulose | 8.50 |
| High viscosity PEO | 62.50 |
| Butylhydroxytoluene | 0.25 |
| Mg stearate | 1.25 |
| Colloidal silica | 1.25 |
| Total tablet core | 250.00 |

### b) Manufacturing procedure

A triturate of tamsulosin hydrochloride, microcrystalline cellulose and medium viscosity hydroxypropylcellulose in the ratio 1 : 40.25 : 8.75 was prepared by mixing all ingredients in a plastic bag.

The obtained triturate was mixed for 5 min. with the remaining part of microcrystalline cellulose in a high shear granulator GRAL PRO 25 (impeller speed 100 rpm). This mixture was granulated with water (high shear granulator GRAL PRO 25, addition of water 58 ml/min., impeller speed 100 rpm, chopper speed slow). The mixture was additionally granulated (high shear granulator GRAL PRO 25, impeller speed 150 rpm, chopper speed slow, time 5 min.). The wet granulate was dried in a fluid bed dryer (Glatt GPCG 3, inlet air temperature 60 °C). When the temperature of the product reached 45 °C, the granulate was sieved through a sieve with mesh size 1.200 mm to break down larger agglomerates and transferred back to the fluid bed dryer. After the drying was completed, the granulate was milled with hammer mill through a sieve with mesh size 0.500 mm (Fitzmill D6).

**Particle size distribution (Sieve analysis)**

| Sieve (µm) | Retained above (%) |
|---|---|
| 400 | 3.27 |
| 315 | 0.80 |
| 200 | 4.46 |
| 125 | 7.46 |
| 100 | 7.59 |
| 45 | 31.98 |
| under 45 | 44.43 |

Then the prepared granulate was mixed with other excipients by gradually adding individual excipients (Erweka). After the addition of excipients, the mixture was blended for 5 min. The final mixture was compressed on a rotary compressing machine Kilian LX 18.

### c) Characterization of the produced tablets

**Intrabatch content uniformity**

| | mg / tablet | % of declared dose |
|---|---|---|
| Beginning of the tabletting: | 0.4005 | 100.1 |
| Middle of the tabletting | 0.4010 | 100.3 |
| End of the tabletting: | 0.3981 | 99.5 |

### EXAMPLE 3

Preparation of coated controlled release tamsulosin hydrochloride tablets

### a) Coated tablet composition

| | mg / tablet |
|---|---|
| Tablet core (e.g. example 2) | 250.00 |
| Hydroxypropylmethylcellulose | 3.261 |
| Hydroxypropylcellulose | 0.815 |
| Polyethylene glycol | 0.612 |
| Titanium dioxide | 0.250 |
| Talc | 0.312 |
| Quinoline yellow | 0.950 |
| Carmine red | 0.040 |
| Black iron oxide | 0.010 |
| Total coated tablet | 256.250 |

### b) Manufacturing procedure

All ingredients were dispersed in water and mixed with Ultra turrax until homogenized dispersion was obtained.
600 g of tamsulosin tablet cores were coated in O'Hara laboratory pan coater until 6.25 mg of coating was applied.

## Claims

1. A pharmaceutical composition of controlled release tamsulosin hydrochloride **characterized in that** it comprises hydroxypropylcellulose and polyethylene oxide.

2. The pharmaceutical composition according to claim 1 **characterized in that** said hydroxypropylcellulose comprises medium viscosity and high viscosity hydroxypropylcellulose.

3. The pharmaceutical composition according to claim 2 **characterized in that** said medium viscosity hydroxypropylcellulose has the viscosity of 10 - 2000 mPa*s.

4. The pharmaceutical composition according to claim 3 **characterized in that** the amount of said medium viscosity hydroxypropylcellulose comprises 10 - 90 % of the total mass of the tablet.

5. The pharmaceutical composition according to claim 2 **characterized in that** said high viscosity hydroxypropylcellulose has the viscosity of above 2000 mPa*s.

6. The pharmaceutical composition according to claim 5 **characterized in that** the amount of said high viscosity hydroxypropylcellulose comprises 10 - 90 % of the total mass of the tablet.

7. The pharmaceutical composition according to claim 1 **characterized in that** the amount of said polyethylene oxide in one tablet being up to 70 mg.

8. The pharmaceutical composition according to any of the claims 1-7 wherein the cores are coated.

9. A process for the preparation of the pharmaceutical composition according to any of the claims 1-7 **characterized in that** it comprises the following steps:
(i) the preparation of the granulate,
(ii) the milling thereof and
(iii) tabletting of the milled granulate.

10. Use of the pharmaceutical composition according to any of the claims 1-8 for the preparation of a medicament for the prevention or treatment of benign prostatic hyperplasia and other related states or conditions.
